# DEMANDE DE BREVET EUROPEEN

(11) **EP 0 963 744 A1**
(43) Date de publication de la demande: **15.12.1999**
(21) Numéro de dépôt: 99401410.8
(22) Date de dépôt: 10.06.1999
(51) Int. Cl.: A61F 9/06, G02B 27/01

(54) **Casque de soudage avec afficheur d'informations**

(30) Priorité: 12.06.1998 FR 9807432
(71) Demandeur: LA SOUDURE AUTOGENE FRANCAISE, F-75007 Paris (FR)
(72) Inventeur: Briand, Francis, 75010 Paris (FR)
(74) Mandataire: Le Moenner, Gabriel

(57) **Abrégé**

Ensemble de soudage à l'arc électrique comprenant un casque (1) de soudage et un générateur de courant de soudage, une torche de soudage ou un dévidoir de fil fusible, ledit casque de soudage (1) comportant des moyens de protection (6) faciaux comprenant une fenêtre équipée de moyens d'atténuation (2) susceptibles d'atténuer au moins une partie du rayonnement émis par un arc électrique, des moyens de maintien (3), des moyens d'affichage (4,5) d'au moins une information, et un système optique (8,8a,8b) relié auxdits moyens d'affichage (4,5). De plus, le casque (1) est muni de moyens de réception d'informations (19) permettant de recevoir des informations émises par des moyens d'émission d'information (20) équipant le générateur (15), la torche (16) et/ou le dévidoir (17). Utilisation d'un tel ensemble dans une opération de soudage, de coupage ou de traitement thermique, mettant en oeuvre un arc électrique.

## Description

La présente invention concerne un ensemble de soudage à l'arc électrique comprenant, d'une part, un casque de soudage et, d'autre part, un générateur de courant de soudage, une torche de soudage et/ou un dévidoir de fil fusible, ledit casque de soudage permettant une visualisation d'informations diverses par l'opérateur durant une opération de soudage à l'arc électrique, lesdites informations étant issues ou transmises par le générateur de courant de soudage, la torche de soudage et/ou le dévidoir de fil fusible

Habituellement, un casque de soudage, encore appelé masque de soudage, comprend des moyens de protection faciaux permettant de protéger le visage ou une partie du visage de l'opérateur d'éventuelles projections de matériaux, lesdits moyens de protection faciaux étant de munis de moyens de maintien permettant de maintenir le casque de soudage en position sur la tête de l'opérateur.

La visualisation de la scène de soudage par l'opérateur se fait au travers d'une fenêtre aménagée au sein des moyens de protection faciaux, laquelle fenêtre est équipée, en général, d'un verre teinté permettant d'atténuer les rayonnements émis par l'arc électrique lors de l'opération de soudage proprement dite.

Il existe plusieurs grades de verres teintés permettant d'obtenir une atténuation plus ou moins importante des rayonnements lumineux; le grade à utiliser dépendant donc de la tâche à accomplir et augmentant avec l'intensité du soudage.

En outre, ce type de casque de soudage équipé de verres teintés permet à l'opérateur, d'une part, de visualiser l'arc et le bain de soudage et, d'autre part, de protéger ledit opérateur du rayonnement lumineux émis par l'arc électrique, à savoir des rayonnements ultraviolets, infrarouges et/ou visibles.

Il existe également des casques à cristaux liquides pour lesquels le verre teinté est remplacé par une cassette composée d'une matrice de cristaux liquide prise en sandwich entre deux verres polarisant, ce qui présente l'avantage de permettre un réglage, soit automatique, soit manuel, du niveau d'atténuation désiré.

Durant une opération de soudage, l'opérateur a besoin d'un certain nombre d'informations issues notamment du générateur de soudage, de la torche de soudage et/ou du dévidoir de fil fusible, et ce, afin de réaliser un soudage efficace.

Or, actuellement, un opérateur équipé d'un casque de soudage classique, tel un casque de soudage à verre teinté ou à matrice de cristaux liquides, doit, lorsqu'il souhaite avoir accès auxdites informations, interrompre son opération de soudage et aller consulter les afficheurs équipant les différents matériels, en prenant soin, au préalable, d'enlever complètement son casque ou au moins de soulever la visière protectrice anti-projections formant les moyens de protection faciaux dudit casque.

En effet, de par l'atténuation importante qu'ils occasionnent, l'opérateur ne peut distinguer à travers ledit verre teinté ou ladite matrice à cristaux liquides que l'arc électrique de soudage et ses abords immédiats.

En d'autres termes, l'atténuation du verre ou de la matrice ne permettent pas à l'opérateur de visualiser directement les afficheurs des différents matériels.

En outre, le fait de devoir interrompre fréquemment l'opération de soudage engendre des pertes de temps, donc une augmentation intempestive des coûts du soudage et une baisse de productivité.

Par ailleurs, le document US-A-2,045,802 décrit un masque de soudage comprenant des moyens visuels, à savoir des ampoules électriques, installés à l'intérieur du masque et destinés à indiquer la longueur de l'arc à l'opérateur.

Par ailleurs, le document EP-A-821257 enseigne un système portable d'affichage d'informations comprenant un dispositif de réception d'images sans fil et un dispositif d'agrandissement et d'affichage d'images sur un écran de visualisation.

En outre, le document DE-A-3136569 décrit un masque de soudage comprenant une commande à distance de poste de soudage et le document US-A-5,343,313 enseigne un système de protection des yeux d'un utilisateur utilisable, en particulier, dans les aéronefs ou les autres véhicules.

Le but de la présente invention est alors de pallier les problèmes suscités, en proposant un casque de soudage susceptible de permettre à l'opérateur d'avoir accès, durant l'opération de soudage, aux différentes informations issues des divers matériels utilisés, tels le générateur, la torche ou le dévidoir, et ne nécessitant pas de retrait, même partiel, dudit casque.

En d'autres termes, la présente invention vise à proposer un ensemble amélioré comprenant à la fois un casque et un générateur de soudage, une torche de soudage et/ou un dévidoir de fil de soudage.

La présente invention concerne alors un ensemble de soudage à l'arc électrique comprenant un casque (1) de soudage et un générateur de courant de soudage, une torche de soudage et/ou un dévidoir de fil fusible, ledit casque de soudage (1) comportant :
- des moyens de protection (6) faciaux comprenant une fenêtre équipée de moyens d'atténuation (2) susceptibles d'atténuer au moins une partie du rayonnement émis par un arc électrique,
- des moyens de maintien (3),
- des moyens d'affichage (4,5) d'au moins une information, et
- au moins un système optique (8, 8a, 8b) relié auxdits moyens d'affichage (4,5),
et ledit casque (1) étant muni, en outre, de moyens de réception d'information permettant de recevoir au moins une information susceptible d'être émise par des moyens d'émission d'information équipant ledit générateur, ladite torche et/ou ledit dévidoir.

Les moyens de protection faciaux du casque sont principalement composés d'une paroi faciale formant écran protecteur, de manière à protéger l'opérateur de toute projection intempestive de métal fondu par exemple.

En outre, les moyens de maintien du casque sont habituellement constitués d'une armature, par exemple en matière plastique, ou de tout moyen analogue permettant de maintenir le casque de soudage, en position sur la tête de l'opérateur.

Dans le cadre de la présente invention, on entend par information, toute donnée exploitable et/ou utile à l'opérateur, laquelle donnée ou information peut être représentée par des caractères alphanumériques, des signes, des dessins ou logos divers, ou tout graphisme similaire.

De même, dans le cadre de l'invention, on utilise indifféremment les termes "casque" ou "masque" et le terme "soudage" revêt un sens général, c'est-à-dire qu'il est employé pour désigner une opération de soudage proprement dit, mais aussi pour toute opération de coupage, de traitement thermique ou analogue, lorsque l'on parle de masque ou casque de soudage.

Selon le cas, le casque de l'ensemble de la présente invention peut comprendre l'une ou plusieurs des caractéristiques suivantes:
- le système optique est agencé de manière à permettre une visualisation efficace d'au moins une information affichée sur lesdits moyens d'affichage;
- les moyens d'affichage comprennent au moins un écran à cristaux liquides ou à diodes;
- le système optique comprend un ou plusieurs miroirs ou prismes de renvoi d'image;
- au moins un prisme ou un miroir est agencé à l'intérieur du casque de soudage dans ou à proximité immédiate du champ de vision de l'opérateur;
- les moyens d'affichage de l'image sont agencés sur ou à l'intérieur du casque de soudage, de préférence hors du champ de vision directe de l'opérateur;
- le système optique est agencé au moins partiellement à l'intérieur du casque de soudage.

L'invention concerne également l'utilisation d'un casque selon l'invention dans un procédé de soudage, de coupage ou de traitement thermique mettant en oeuvre un arc électrique et nécessitant l'usage d'un tel ensemble de soudage, notamment une opération de soudage TIG (Tungsten Inert Gas), MIG (Metal Inert Gas), MAG (Metal Active Gas), ou de soudage plasma ou à l'électrode, ou une opération de coupage plasma ou d'oxycoupage.

L'invention va maintenant être décrite plus en détail à l'aide de figures données à titre illustratif, mais non limitatif de l'invention.

La figure 1 représente un schéma d'un opérateur 10 muni d'une torche de soudage 11 à l'arc électrique 12, lequel opérateur 10 est équipé d'un casque 1 selon l'art antérieur, c'est-à-dire un casque 1 de soudage muni de moyens de protection faciaux 6 et d'une fenêtre 2 équipée d'un verre teinté permettant d'atténuer une partie du rayonnement émis par l'arc électrique 12.

La figure 2 représente, quant à elle, un casque de soudage d'un ensemble selon la présente invention.

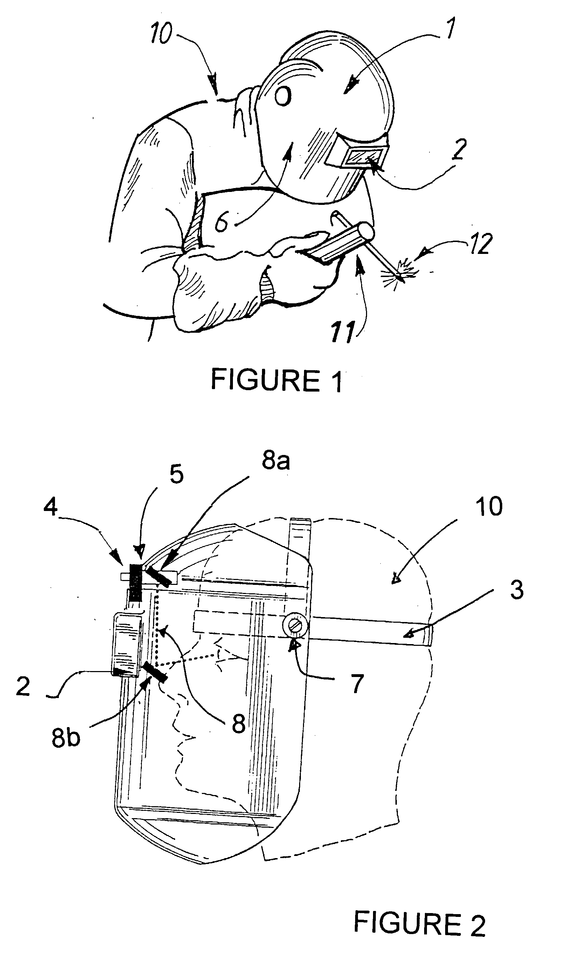

Plus précisément, la figure 2 représente un opérateur 10 équipé d'un casque de soudage 1 équipé de moyens de maintien 3 permettant de maintenir le casque de soudage en position sur la tête de l'utilisateur 10.

Dans ce cas, les moyens de maintien 3 comprennent essentiellement une armature constituée de sangles reliées par des vis, des rivets ou autres moyens analogues 7 aux moyens de protection faciaux 6.

Les moyens de protection faciaux 6 sont constitués principalement d'un écran protecteur 6, en matière plastique par exemple, sur lequel ont été fixés des moyens d'affichage d'informations 4, par exemple un ou plusieurs écrans 5 à cristaux liquides ou écrans LCD (Liquid Crystal Display) ou à diodes lumineuses ou LED (Light Emitting Diode).

Afin de permettre à l'opérateur de visualiser correctement le ou les écrans 5, il est généralement nécessaire d'adjoindre aux écrans 5 un système optique 8 de transfert de l'image, tels, par exemple, un ou plusieurs miroirs ou prismes, susceptibles de permettre à l'opérateur une visualisation correcte et rapide de ou des informations dispensées par l'écran 5.

Selon l'invention, la lecture desdites informations est très rapide et peut être effectuée d'un simple coup d'oeil par l'opérateur.

En effet, il est indispensable, pour permettre une lecture desdites informations d'éloigner artificiellement l'oeil de l'opérateur de l'écran 5 afin que la distance oeil/afficheur soit supérieure au punctum proximum de l'oeil, c'est-à-dire la distance minimale devant être respectée pour que l'opérateur soit capable de lire les' informations affichées sur l'écran 5.

Plus précisément, comme représenté sur la figure 2, les moyens d'affichage 4 d'informations sont agencés sur la partie supérieure du casque de soudage, hors du champ de vision normal de l'opérateur.

Un premier miroir 8a de renvoi permet de réfléchir l'image dispensée par l'écran 5 en la renvoyant vers un second miroir de renvoi 8b.

Le second miroir de renvoi 8b est placé dans le champ de vision de l'opérateur ou à la périphérie de celui-ci, de sorte que l'opérateur ne soit pas gêné par ce second miroir 8b de renvoi pendant le soudage proprement dit, c'est-à-dire lorsque l'opérateur est concentré sur l'arc électrique et le joint de soudure en cours de réalisation, mais qu'il puisse lire les informations qui s'y réfléchissent et ce, d'un simple coup d'oeil, de préférence vers le bas.

En fait, comme expliqué ci-avant, l'utilisation des deux miroirs de renvoi 8a et 8b permet d'allonger la distance oeil/écran 5 et donc aussi de rendre possible la lecture des informations s'affichant sur cet écran 5.

En outre, le second miroir 8b présente l'avantage de redresser l'image inversée par le premier miroir 8a de renvoi.

Toutefois, la présente invention n'est pas limitée aux miroirs, en fait tout autre système de renvoi d'image permettant d'accroître la distance oeil/écran 5 peut être utilisé, en particulier un système à prismes.

Par ailleurs, la liaison entre le casque de soudage et plus précisément entre les moyens d'affichage 4 de l'information et les différents matériels mis en oeuvre durant l'opération de soudage (générateur, torche...) peut être aérienne, par exemple de type infrarouge, à fréquence modulée, haute fréquence ou filière (liaison par câble).

Dans le cas d'une liaison aérienne, on peut placer l'émetteur sur la torche de soudage, par exemple, et le récepteur sur le casque de soudage.

En outre, les informations dispensées par l'écran 5 peuvent être, par exemples, soit des paramètres de soudage, tels la tension, l'intensité du courant, la vitesse d'avance du fil..., soit des informations sur l'environnement, telles l'heure ou la température, soit des informations relatives au cordon de soudure en cours de réalisation, par exemple la conformité des paramètres de soudage, aux spécifications de réalisation d'une opération de soudage donnée, ou des modifications ou conseils à suivre pour optimiser la soudure, tels les régimes de transfert à adopter.

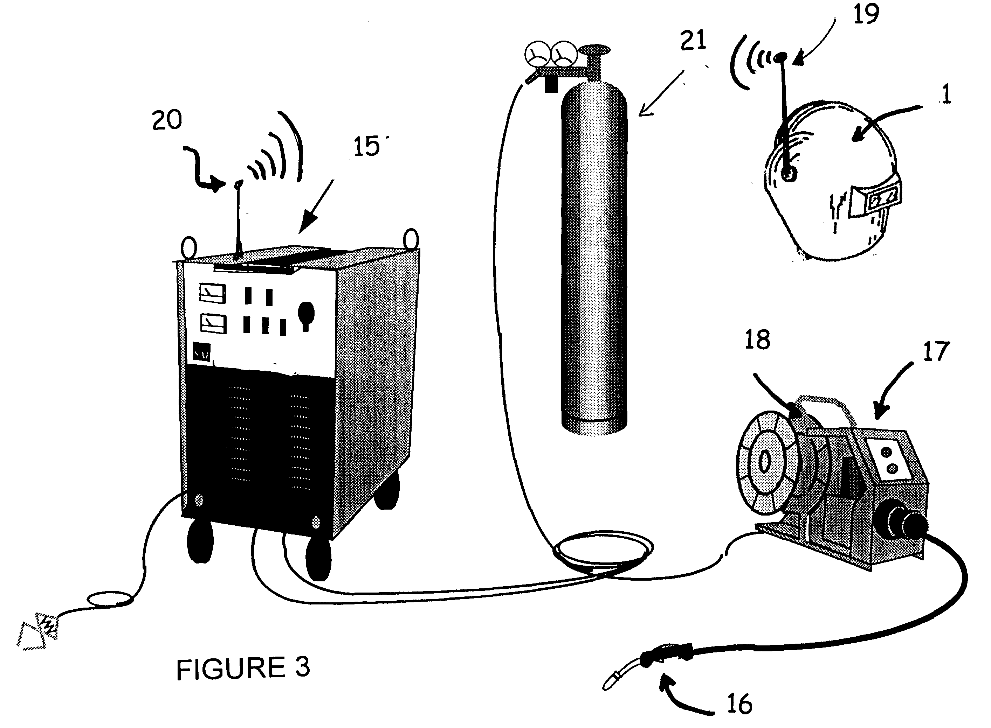

La figure 3 représente, quant à elle, un ensemble de soudage à l'arc électrique selon l'invention comprenant un ensemble de soudage à l'arc électrique comprenant un casque de soudage 1 selon l'invention et un générateur 15 de courant de soudage, une torche 16 de soudage alimentée en gaz par une bouteille 21 de gaz sous pression et un dévidoir 17 de fil 18 fusible, ledit casque de soudage 1 étant muni de moyens de réception d'informations permettant de recevoir au moins une information susceptible d'être émise par des moyens d'émission d'information équipant ledit générateur 15, ladite torche 16 et/ou ledit dévidoir 17.

Ici, les moyens de réception comprennent au moins une antenne réceptrice 19 portée par ledit casque 1 et les moyens d'émission 20 d'informations comprennent au moins une antenne émettrice 20 portée par ledit générateur 15.

Bien entendu, avant leur émission vers le casque 1 de soudage, les informations issues du générateur 15, de la torche 16 et/ou du dévidoir 17 peuvent subir des prétraitements, tels des mesures, des calculs, des définitions....

Le casque de soudage selon l'invention peut être utilisé dans toute opération de soudage, en particulier à l'arc électrique.

Sur la figure 3, les moyens d'émission et les moyens de réception d'informations ne sont pas détaillés.

En variante, le casque de soudage 1 peut être relié au générateur 15 au dévidoir 17 et/ou à la torche 16 par l'intermédiaire de câbles de connexion ou autres moyens analogues.

## Revendications

1. Ensemble de soudage à l'arc électrique comprenant un casque (1) de soudage et un générateur de courant de soudage, une torche de soudage et/ou un dévidoir de fil fusible, ledit casque de soudage (1) comportant :
- des moyens de protection (6) faciaux comprenant une fenêtre équipée de moyens d'atténuation (2) susceptibles d'atténuer au moins une partie du rayonnement émis par un arc électrique,
- des moyens de maintien (3),
- des moyens d'affichage (4,5) d'au moins une information, et
- au moins un système optique (8, 8a, 8b) relié auxdits moyens d'affichage (4,5),
et ledit casque (1) étant muni, en outre, de moyens de réception d'information permettant de recevoir au moins une information susceptible d'être émise par des moyens d'émission d'information équipant ledit générateur, ladite torche et/ou ledit dévidoir.

2. Ensemble selon la revendication 1, caractérisé en ce que le système optique (8a, 8b) dudit casque est agencé de manière à permettre une visualisation efficace d'au moins une information affichée sur lesdits moyens d'affichage (4,5).

3. Ensemble selon l'une des revendications 1 ou 2, caractérisé en ce que les moyens d'affichage (4,5) dudit casque comprennent au moins un écran à cristaux liquides ou à diodes.

4. Ensemble selon l'une des revendications 1 à 3, caractérisé en ce que le système optique (8a, 8b) dudit casque comprend un ou plusieurs miroirs ou prismes de renvoi d'image.

5. Ensemble selon la revendication 4, caractérisé en ce qu'au moins un prisme ou un miroir (8b) est agencé à l'intérieur du casque dans ou à proximité immédiate du champ de vision de l'opérateur.

6. Ensemble selon l'une des revendications 1 à 5, caractérisé en ce que les moyens d'affichage (4, 5) de l'image sont agencés sur ou à l'intérieur du casque (1), de préférence hors du champ de vision directe de l'opérateur.

7. Ensemble selon l'une des revendications 1 à 6, caractérisé en ce que le système optique (8, 8a, 8b) est agencé au moins partiellement à l'intérieur du casque de soudage (1).

8. Ensemble selon la revendication 8, caractérisé en ce que les moyens de réception dudit casque comprennent au moins une antenne réceptrice portée par ledit casque (1) et/ou en ce que les moyens d'émission d'information comprennent au moins une antenne émettrice portée par ledit générateur, ladite torche et/ou ledit dévidoir.

9. Casque (1) susceptible de constituer une partie d'un ensemble selon l'une des revendications 1 à 8, comportant des moyens de protection (6) faciaux comprenant une fenêtre équipée de moyens d'atténuation (2) susceptibles d'atténuer au moins une partie du rayonnement émis par un arc électrique, des moyens de maintien (3), des moyens d'affichage (4,5) d'au moins une information, et au moins un système optique (8, 8a, 8b) relié auxdits moyens d'affichage (4,5), ledit casque (1) comprenant, en outre, des moyens de réception d'information permettant de recevoir au moins une information susceptible d'être émise par des moyens d'émission d'information équipant un générateur, une torche et/ou un dévidoir de fil.

10. Utilisation d'un ensemble de soudage selon l'une des revendications 1 à 8 dans une opération de soudage, de coupage ou de traitement thermique, mettant en oeuvre un arc électrique.
